# EUROPÄISCHE PATENTANMELDUNG

(11) **EP 0 936 270 A2**
(43) Veröffentlichungstag der Anmeldung: **18.08.1999**
(21) Anmeldenummer: 99102357.3
(22) Anmeldetag: 06.02.1999
(51) Int. Cl.: C12N 9/64, C12N 15/55, C07K 16/40, A61K 38/48, A61K 48/00, C12Q 1/68, G01N 33/573

(54) **Serinprotease aus der Prostata**

(30) Priorität: 12.02.1998 DE 19805633
(71) Anmelder: BASF AKTIENGESELLSCHAFT, 67056 Ludwigshafen (DE)
(72) Erfinder: Kröger, Burkhard, Dr., 67117 Limburgerhof (DE)

(57) **Zusammenfassung**

Neue Serinproteasen aus humaner Prostata, dafür codierendes Gen und ihre Verwendung.

SEQUENCE LISTING

<110> BASF Aktiengesellschaft

<120> Neue Serinprotease aus humaner Prostata

<130> OZ 0050/48778

<140>
<141>

<150> DE 19805633.8
<151> 1998-02-12

<160> 2

<170> PatentIn Ver. 2.0

<210> 1
<211> 1369
<212> DNA
<213> Homo sapiens

<220>
<221> CDS
<222> (2)..(694)

<400> 1
   c gtc tct ggt agc tgc agc caa atc ata aac ggc gag gac tgc agc ccg 49
     Val Ser Gly Ser Cys Ser Gln Ile Ile Asn Gly Glu Asp Cys Ser Pro
       1               5                  10                  15
   cac tcg cag ccc tgg cag gcg gca ctg gtc atg gaa aac gaa ttg ttc    97
   His Ser Gln Pro Trp Gln Ala Ala Leu Val Met Glu Asn Glu Leu Phe
                20                  25                  30
   tgc tcg ggc gtc ctg gtg cat ccg cag tgg gtg ctg tca gcc gca cac    145
   Cys Ser Gly Val Leu Val His Pro Gln Trp Val Leu Ser Ala Ala His
            35                  40                  45
   tgt ttc cag aac tcc tac acc atc ggg ctg ggc ctg cac agt ctt gag    193
   Cys Phe Gln Asn Ser Tyr Thr Ile Gly Leu Gly Leu His Ser Leu Glu
        50                  55                  60
   gcc gac caa gag cca ggg agc cag atg gtg gag gcc agc ctc tcc gta    241
   Ala Asp Gln Glu Pro Gly Ser Gln Met Val Glu Ala Ser Leu Ser Val
    65                  70                  75                  80
   cgg cac cca gag tac aac aga ccc ttg ctc gct aac gac ctc atg ctc    289
   Arg His Pro Glu Tyr Asn Arg Pro Leu Leu Ala Asn Asp Leu Met Leu
                    85                  90                  95
   atc aag ttg gac gaa tcc gtg tcc gag tct gac acc atc cgg agc atc 337
   Ile Lys Leu Asp Glu Ser Val Ser Glu Ser Asp Thr Ile Arg Ser Ile
   100 105 110
   agc att gct tcg cag tgc cct acc gcg ggg aac tct tgc ctc gtt tct 385
   Ser Ile Ala Ser Gln Cys Pro Thr Ala Gly Asn Ser Cys Leu Val Ser
   115 120 125
   ggc tgg ggt ctg ctg gcg aac ggc aga atg cct acc gtg ctg cag tgc 433
   Gly Trp Gly Leu Leu Ala Asn Gly Arg Met Pro Thr Val Leu Gln Cys
   130 135 140
   gtg aac gtg tcg gtg gtg tct gag gag gtc tgc agt aag ctc tat gac 481
   Val Asn Val Ser Val Val Ser Glu Glu Val Cys Ser Lys Leu Tyr Asp
   145 150 155 160
   ccg ctg tac cac ccc agc atg ttc tgc gcc ggc gga ggg caa gac cag 529
   Pro Leu Tyr His Pro Ser Met Phe Cys Ala Gly Gly Gly Gln Asp Gln
   165 170 175
   aag gac tcc tgc aac ggt gac tct ggg ggg ccc ctg atc tgc aac ggg 577
   Lys Asp Ser Cys Asn Gly Asp Ser Gly Gly Pro Leu Ile Cys Asn Gly
   180 185 190
   tac ttg cag ggc ctt gtg tct ttc gga aaa gcc ccg tgt ggc caa gtt 625
   Tyr Leu Gln Gly Leu Val Ser Phe Gly Lys Ala Pro Cys Gly Gln Val
   195 200 205
   ggc gtg cca ggt gtc tac acc aac ctc tgc aaa ttc act gag tgg ata 673
   Gly Val Pro Gly Val Tyr Thr Asn Leu Cys Lys Phe Thr Glu Trp Ile
   210 215 220
   gag aaa acc gtc cag gcc agt taactctggg gactgggaac ccatgaaatt 724
   Glu Lys Thr Val Gln Ala Ser
   225 230
   gacccccaaa tacatcctgc ggaaggaatt caggaatatc tgttcccagc ccctcctccc 784
   tcaggcccag gagtccaggc ccccagcccc tcctccctca aaccaagggt acagatcccc 844
   agcccctcct ccctcagacc caggagtcca gaccccccag cccctcctcc ctcagaccca 904
   ggagtccagc ccctcctccc tcagacccag gagtccagac cccccagccc ctcctccctc 964
   agacccaggg gtccagcctc tcctccctca gacccaggag tccagacccc ccagcccctc 1024
   ctccctcaga cccaggagtc cagcccctcc tccctcagac ccaggagtcc agatccccca 1084
   gcccctcctc cctcagaccc aggggtccag gcccccaacc cctcctccct cagactcaga 1144
   ggtccaagcc cccaacccct ccttccccag acccagaggt ccaggtacca gcccctcctc 1204
   cctcagaccc agcggtccaa tgccacctag actctccctg tacacagtgc ccccttgtgg 1264
   cacgttgacc caaccttacc agttggtttt tcattttttg tccctttccc ctagatccag 1324
   aaataaagtc taagagaagc gcaaaaaaaa aaaaaaaaaa aaaaa 1369

<210> 2
<211> 231
<212> PRT
<213> Homo sapiens

<400> 2
Val Ser Gly Ser Cys Ser Gln Ile Ile Asn Gly Glu Asp Cys Ser Pro
1               5                   10                  15

His Ser Gln Pro Trp Gln Ala Ala Leu Val Met Glu Asn Glu Leu Phe
            20                  25                  30

Cys Ser Gly Val Leu Val His Pro Gln Trp Val Leu Ser Ala Ala His
        35                  40                  45

Cys Phe Gln Asn Ser Tyr Thr Ile Gly Leu Gly Leu His Ser Leu Glu
    50                  55                  60

Ala Asp Gln Glu Pro Gly Ser Gln Met Val Glu Ala Ser Leu Ser Val
65                  70                  75                  80

Arg His Pro Glu Tyr Asn Arg Pro Leu Leu Ala Asn Asp Leu Met Leu
                85                  90                  95

Ile Lys Leu Asp Glu Ser Val Ser Glu Ser Asp Thr Ile Arg Ser Ile
            100                 105                 110

Ser Ile Ala Ser Gln Cys Pro Thr Ala Gly Asn Ser Cys Leu Val Ser
        115                 120                 125

Gly Trp Gly Leu Leu Ala Asn Gly Arg Met Pro Thr Val Leu Gln Cys
    130                 135                 140

Val Asn Val Ser Val Val Ser Glu Glu Val Cys Ser Lys Leu Tyr Asp
145                 150                 155                 160

Pro Leu Tyr His Pro Ser Met Phe Cys Ala Gly Gly Gly Gln Asp Gln
                165                 170                 175

Lys Asp Ser Cys Asn Gly Asp Ser Gly Gly Pro Leu Ile Cys Asn Gly
            180                 185                 190

Tyr Leu Gln Gly Leu Val Ser Phe Gly Lys Ala Pro Cys Gly Gln Val
     195                 200                 205

Gly Val Pro Gly Val Tyr Thr Asn Leu Cys Lys Phe Thr Glu Trp Ile
    210                 215                 220

Glu Lys Thr Val Gln Ala Ser
225                 230

## Beschreibung

Die vorliegende Erfindung betrifft neue Serinproteasen aus humaner Prostata, deren Gene und Verwendung.

Serinproteasen aus der Prostata sind bereits bekannt. Sie erfüllen dort u.a. die Aufgabe, durch proteolytischen Verdau der Semenogeline I und II, sowie des Fibronectins die Samenflüssigkeit zu verflüssigen und die Spermienmotilität zu erhöhen. Auch die Spaltung des Insulin-like growth factor/binding protein-3 und damit einhergehende mitogene Wirkung des Wachstumsfaktors wird prostataspezifischen Proteasen zugeschrieben.

Prominentester Vertreter ist das Prostataspezifische Antigen (PSA), ein Mitglied der menschlichen Gewebekallikreinfamilie. PSA hat in den letzten Jahren eine große Bedeutung als diagnostischer Marker von Prostataerkrankungen erlangt. Die Aussagekraft des PSA Markers ist aber limitiert, da PSA-Spiegel nicht eindeutig zwischen benigner Prostatahyperplasie und Prostatatumoren unterscheiden. Außerdem können auch bakterielle Prostatitiden und andere Erkrankungen den Serum-PSA-Spiegel erhöhen. Des weiteren wird der Serum-PSA-Spiegel zur Erfolgskontrolle nach Prostatektomie eingesetzt.

Gegenstand der Erfindung ist ein isoliertes Protein, enthaltend die in SEQ ID NO:2 dargestellte Aminosäuresequenz oder eine daraus durch Substitution, Insertion oder Deletion von einem oder mehreren Aminosäureresten erhältliche Sequenz, wobei wenigstens noch eine der wesentlichen biologischen Eigenschaften des in SEQ ID NO:2 dargestellten Proteins erhalten bleibt.

Das erfindungsgemäße Protein gemäß SEQ ID NO:2 ist eine Serinprotease. Es handelt sich um ein neues Mitglied der Serinproteasefamilie EC 3.4.21. Sequenzvergleiche zeigen einen hohen Verwandtschaftsgrad mit Chymotrypsinogen und verschiedenen Kallikreinen.

Ein weiterer Gegenstand der Erfindung sind Proteine vom Serin-Protease-Typ, die ausgehend von der in SEQ ID NO:2 dargestellten Aminosäuresequenz durch gezielte Veränderungen herstellbar sind. Beispielsweise können bestimmte Aminosäuren durch solche mit ähnlichen physikochemischen Eigenschaften (Raumerfüllung, Basizität) ersetzt werden. Es können aber auch ein oder mehrere Aminosäuren hinzugefügt oder entfernt werden, oder mehrere dieser Maßnahmen miteinander kombiniert werden. Die solchermaßen gegenüber der SEQ ID NO:2 veränderten Proteine besitzen wenigstens 60%, bevorzugt wenigstens 75% Homologie zu SEQ ID NO:2, berechnet nach dem Algorithmus von Pearson und Lipman, Proc. Natl. Acad. Sci (USA) 85, 2444-2448.

Ein weiterer Gegenstand der Erfindung sind Nukleinsäuresequenzen, die für die oben beschriebenen Proteine codieren, insbesondere solche mit der in SEQ ID NO:1 dargestellten Primärstruktur.

Die Nukleotidsequenz mit der in SEQ ID NO:1 dargestellten Struktur wurde ursprünglich in mehreren cDNA-Bibliotheken aus humaner Prostata identifiziert. Die Analyse der Verteilung der zugehörigen mRNA in 50 verschiedenen menschlichen Geweben ergab fast ausschließliche Expression in der Prostata. Das Transkript läßt sich in sehr geringem Umfang aber auch in Niere, Speicheldrüse, Schilddrüse und Nebenniere nachweisen.

Das durch die vorliegende cDNA kodierte Polypeptid läßt sich zweifelsfrei als Serinprotease identifizieren. Die katalytische Triade, das Kennzeichen dieser Proteinfamilie findet sich in jeweils sehr konservierten Aminosäuresequenzabschnitten mit den Positionen Histidin 48, Aspartat 93 und Serin 184 in SEQ ID NO:2. Die größte Verwandtschaft auf Aminosäureebene findet sich mit 47,1 % Identität zu humanem Chymotrypsinogen des Stratum corneum (FastA-Programm, Pearson und Lipman, Proc. Natl. Acad. Sci (USA) 85, 2444-2448).

Der vorliegenden Sequenz fehlt der für den N-Terminus kodierende Bereich der cDNA. Aufgrund der zu beobachtenden Kolinearität der Sequenzhomologien zwischen SEQ ID NO:2 und anderen Serinproteasen, besonders humanes Chymotrypsinogen aus Stratum corneum läßt sich ableiten, daß der fehlende Teil ein Signalpeptid von mindestens 10 Aminosäuren und einen Proproteinanteil umfaßt.

Wie bei vielen anderen Vertretern dieser Proteasefamilie wird ein inaktives Proprotein oder Zymogen von der Zelle gebildet, und es bedarf eines proteolytischen Verdaues N-terminaler Aminosäuren durch spezifische oder unspezifische Proteasen, um zum reifen Protein aktiviert zu werden. Eine Spaltstelle für diesen proteolytischen Schritt befindet sich zwischen Aminosäure 7 und 8 in SEQ ID NO:2 (nach Gln 7).

Die vorliegende cDNA kann durch dem Fachmann geläufige Klonierungs- und Transfektionsmethoden in verschiedenen Expressionssystemen zur Expression gebracht werden. Dies sind beispielsweise pro- oder eukaryotische Vektorsysteme, wie SV40, CMV, Baculovirus, Adenovirus; Plasmide; Phagemide, Phagen.

Dazu wird die erfindungsgemäße Nukleinsäuresequenz üblicherweise mit genetischen Regulationselementen wie Transkriptions- und Translationssignalen funktionell verknüpft. Mit den solchermaßen hergestellten rekombinanten Nukleinsäurekonstrukten werden anschließend Wirtsorganismen transformiert.

Eine bevorzugte Ausführungsform ist die Verknüpfung der erfindungsgemäßen Nukleinsäuresequenz mit einem Promotor, wobei der Promotor 5'-upstream zu liegen kommt. Weitere Regulationssignale wie Terminatoren, Polyadenylierungssignale, Enhancer können in dem Nukleinsäurekonstrukt Anwendung finden.

Als Wirtszellen sind Bakterien wie Escherichia coli, eukaryotische Mikroorganismen wie Saccharomyces cerevisiae, höhere eukaryotische Zellen aus Tieren oder Pflanzen, beispielsweise Sf9 oder CHO-Zellen, geeignet.

Gewünschtenfalls kann das Gen auch in transgenen Organismen wie transgenen Tieren, z.B. Mäusen, Schafen oder transgenen Pflanzen zur Expression gebracht werden.

Die beschriebene Serinprotease kann dazu sowohl als Prepro-, als auch Proprotein (Zymogen), als auch als aktive (reife) Protease exprimiert werden.

Darüberhinaus kann das Genprodukt auch in Form therapeutisch oder diagnostisch geeigneter Fragmente exprimiert werden. Zur Isolation des rekombinanten Proteins können Vektorsysteme oder Oligonukleotide verwendet werden, die die cDNA um bestimmte Nukleotidsequenzen verlängern und damit für veränderte Polypeptide kodieren, die einer einfacheren Reinigung dienen. Als solche "Tags" sind in der Literatur z.B. Hexa-Histidin-Anker bekannt oder Epitope, die als Antigene verschiedener Antikörper erkannt werden können (beschrieben zum Beispiel in Harlow, E. and Lane, D. (1988) Antibodies: A Laboratory Manual. Cold Spring Harbor (N.Y.) Press).

Ausgehend von der Peptidsequenz können synthetische Peptide generiert werden, die als Antigene für die Produktion von Antikörpern eingesetzt werden. Es ist auch möglich, das Polypeptid oder Bruchstücke davon zur Generierung von Antikörpern einzusetzen.

Die Herstellung von Antikörpern ist eine dem Fachmann geläufige Tätigkeit. Mit Antikörpern sind sowohl polyklonale, monoklonale, humane oder humanisierte Antikörper oder Fragmente davon, single chain Antikörper oder auch synthetische Antikörper gemeint.

Die vorliegende cDNA bietet außerdem die Voraussetzung, die genomische Sequenz dieses neuen Serinproteasegens zu klonieren. Darunter fällt auch die dazugehörige regulatorische oder Promotorsequenz, die beispielsweise durch Sequenzierung des 5' upstream Bereiches der vorliegenden cDNA zugänglich wird. Die Sequenzinformation der cDNA ist auch die Grundlage für die Herstellung von antisense Molekülen oder Ribozymen.

Eine weitere Möglichkeit des Einsatzes der Nukleotidsequenz oder Teilen davon ist die Erzeugung transgener Tiere. Transgene Überexpression oder genetischer Knockout der Sequenzinformation in geeigneten Tiermodellen kann wertvolle weitere Informationen über die (Patho-)Physiologie der neuen Serinprotease liefern.

Die regulatorischen Sequenzen, insbesondere die Promotorsequenzen oder Teilsequenzen des Promotors dieses Genes können für die gewebespezifische Expression von diesem und weiteren Genen verwendet werden. Damit ergibt sich die Möglichkeit, Prostata-spezifische Genexpression durchzuführen.

In Situationen, in denen ein Mangel an der beschriebenen Proteinaktivität herrscht, können mehrere Methoden zur Substituierung eingesetzt werden. Zum einen kann das Protein, natürlich oder rekombinant, direkt oder durch geeignete Maßnahmen in Form seiner kodierenden Nukleinsäure (d.h. DNA oder RNA) appliziert werden. Dazu können sowohl virale, als auch nichtvirale Vehikel zum Einsatz kommen. Ein weiterer Weg bietet sich durch die Stimulation des endogenen, körpereigenen Genes durch geeignete Substanzen. Solche Substanzen lassen sich beispielsweise auffinden, indem man ihre Wirkung auf die Transkriptionselemente des Serinprotease-Gens ermittelt.

In Situationen, in denen überschüssige Proteinaktivität der beschriebenen Protease vorliegt, können verschiedene Inhibitoren der Proteinaktivität eingesetzt werden. Diese Inhibition kann sowohl durch antisense Moleküle oder Ribozyme, oder Antikörper und Oligonukleotide, als auch durch niedermolekulare Verbindungen erreicht werden.

Weiterhin können die cDNA, die genomische DNA, der Promotor, als auch das Polypeptid, sowie Teilfragmente davon in rekombinanter oder nichtrekombinanter Form zur Ausarbeitung eines Testsystems verwendet werden. Dieses Testsystem ist geeignet, die Aktivität des Promotors oder des Proteins in Anwesenheit einer Testsubstanz zu messen. Bevorzugt handelt es sich dabei um einfache Meßmethoden (colorimetrischer, luminometrischer, fluorimetrischer oder radioaktiver Art,) die die schnelle Meßbarkeit einer Vielzahl von Testsubstanzen erlauben. Die beschriebenen Testsysteme erlauben die Bindung oder Agonisierung oder Antagonisierung von Testsubstanzen in Bezug zur neuen Protease zu beschreiben.

Die Bestimmung von Menge, Aktivität und Verteilung der Protease oder ihrer zugrundeliegenden mRNA im menschlichen Körper kann zur Diagnose, Prädisposition und zum Monitoring bei bestimmten Erkrankungen dienen. Desgleichen kann die Sequenz der cDNA sowie der genomischen Sequenz zu Aussagen über genetische Ursachen und Prädispositionen bestimmter Erkrankungen herangezogen werden. Dazu können sowohl DNA/RNA-Proben, sowie unnatürliche DNA/RNA-Proben, als auch Antikörper verschiedenster Art benutzt werden. Dabei dient die beschriebene Nukleotidsequenz oder Teile davon in Form geeigneter Proben zur Aufdeckung von Punktmutationen oder Deletionen/Insertionen.

Weiterhin kann die beschriebene Proteinaktivität benutzt werden, um ihre natürlichen Substrate zu bestimmen und zu isolieren. Eine geeignete Methode für diese Vorgehensweise wurde beschrieben von: Kothakota, S. et al., Science 278, 294-298 (1997).

Die vorliegende Nukleinsäuresequenz und die von ihr kodierte Protease sowie davon abgeleitete Reagenzien (Oligonukleotide, Antikörper) können zur Diagnose und Therapie von Erkrankungen der Prostata und des "Reproduktionssystems", sowie bei Fertilitätsstörungen, Brustkrebs, Prostatitis, benigner Prostatahyperplasie, Prostatakarzinom, und Metastasen desselben eingesetzt werden.

Außerdem wird die Diagnose und Bestimmung von genetischen Prädispositionen für o.a. Erkrankungen ermöglicht.

Weiterhin kann ein Monitoring von Behandlung und Therapie o.a. Erkrankungen durchgeführt werden.

Ein weiterer Gegenstand der Erfindung ist ein Verfahren zum qualitativen und quantitativen Nachweis einer Nukleinsäure nach Anspruch 3 in einer biologischen Probe, das folgende Schritte umfaßt:
a) Inkubation einer biologischen Probe mit einer bekannten Menge an Nukleinsäure gemäß Anspruch 3 oder einer bekannten Menge an Oligonukleotiden, die als Primer für eine Amplifikation der Nukleinsäure gemäß Anspruch 3 geeignet sind,
b) Nachweis der Nukleinsäure gemäß Anspruch 3 durch spezifische Hybridisierung oder PCR-Amplifikation,
c) Vergleich der Menge an hybridisierender Nukleinsäure gemäß Anspruch 3 oder an durch PCR Amplifikation gewonnener Nukleinsäure gemäß Anspruch 3 mit einem Standard.

Außerdem betrifft die Erfindung ein Verfahren zum qualitativen und quantitativen Nachweis eines Proteins gemäß Anspruch 1 in einer biologischen Probe, das folgende Schritte umfaßt:
a) Inkubation einer biologischen Probe mit einem Antikörper, der spezifisch gegen das Protein gemäß Anspruch 1 gerichtet ist,
b) Nachweis des Antikörper/Antigenkomplexes,
c) Vergleich der Mengen des Antikörper/Antigenkomplexes mit einem Standard.

Als Standard wird üblicherweise eine biologische Probe aus einem gesunden Organismus entnommen.

Inhibitoren der erfindungsgemäßen Proteasen können Verwendung finden zur Behandlung von metastasierenden Tumoren. Ein weiteres Einsatzgebiet dieser Inhibitoren ist die Kontrazeption durch Inhibierung der Spermienreifung.

### Beispiel 1

### Klonierung der Serinprotease cDNA

Bei der Sequenzanalyse von cDNA-Klonen einer cDNA-Bibliothek aus menschlicher Prostata (z.B.Human Prostate 5'-Stretch Plus cDNA, Fa. Clontech, #HL5026t) wurde vorliegende Sequenz SEQ ID NO:1 identifiziert. Die Sequenz dieses Klones umfaßt den kodierenden Bereich ab Aminosäure 1 bis zum Stopkodon und zusätzlich den 3' nichttranslatierten Bereich einschließlich des Polyadenylierungssignals und des Poly(A)-Schwanzes.

### Beispiel 2

### Expression der neuen Serinprotease in menschlichen Geweben

Die Expression der neuen Serinprotease wurde in 50 verschiedenen menschlichen Geweben mittels RNA-Dotblot-Analyse untersucht. Ein Blot der Firma Clontech (#7770-1) wurde dazu mit einer Nukleotidsequenz aus SEQ ID NO: 1, Position 92-466 als Probe hybridisiert. Die Probe wurde durch in vitro Transkription, der entsprechenden cDNA in Anwesenheit Digoxigenin-markierter Nukleotide hergestellt, wie in BioTechniques 13(4), 604-610 (1992) beschrieben.

Nach stringentem Waschen wurde das Transkript hauptsächlich in Prostatagewebe nachgewiesen.

## Patentansprüche

1. Isoliertes Protein, enthaltend die in SEQ ID NO:2 dargestellte Aminosäuresequenz oder eine daraus durch Substitution, Insertion oder Deletion von einem oder mehreren Aminosäureresten erhältliche Sequenz, wobei wenigstens noch eine der wesentlichen biologischen Eigenschaften des in SEQ ID NO:2 dargestellten Proteins erhalten bleibt.

2. Protein nach Anspruch 1, dadurch gekennzeichnet, daß es sich um ein humanes Protein handelt.

3. Nukleinsäuresequenz codierend für ein Protein nach Anspruch 1.

4. Nukleinsäuresequenz nach Anspruch 3, dadurch gekennzeichnet, daß sie für ein Protein codiert, das wenigstens 60% Identität mit der in SEQ ID NO:2 dargestellten Sequenz hat.

5. Nukleinsäuresequenz nach Anspruch 4, dadurch gekennzeichnet, daß sie die in SEQ ID NO:1 dargestellte Sequenz enthält.

6. Rekombinantes Nukleinsäurekonstrukt, enthaltend eine Nukleinsäuresequenz gemäß Anspruch 3 funktionell verknüpft mit mindestens einem genetischen Regulationselement.

7. Wirtsorganismus, transformiert mit einer Nukleinsäuresequenz nach Anspruch 3.

8. Wirtsorganismus, transformiert mit einem rekombinanten Nukleinsäurekonstrukt nach Anspruch 6.

9. Verwendung eines Proteins nach Anspruch 1 zur Identifizierung von spezifischen Enzyminhibitoren.

10. Verwendung eines Proteins nach Anspruch 1 oder eines immunogenen Peptidfragmentes davon als Antigen zur Erzeugung von spezifischen Antikörpern gerichtet gegen Proteine gemäß Anspruch 1.

11. Antikörper, die spezifisch das Protein nach Anspruch 1 erkennen.

12. Verwendung einer Nukleinsäuresequenz nach Anspruch 3 zur Gentherapie.

13. Verwendung einer zu der Sequenz gemäß Anspruch 3 komplementären Nukleinsäuresequenz zur Gentherapie.

14. Verfahren zur Identifizierung von spezifischen Inhibitoren eines Proteins gemäß Anspruch 1 indem man in das Protein gemäß Anspruch 1 mit einem Substrat in Abwesenheit und Anwesenheit einer auf inhibitorische Wirkung zu testenden Substanz (Testsubstanz) inkubiert und anschließend die biologische Aktivität des Proteins in An- und Abwesenheit der Testsubstanz vergleicht.

15. Verfahren zum Auffinden von Substanzen mit spezifischer Bindungsaffinität zu einem Protein nach Anspruch 1, das folgende Schritte umfaßt:
a) Inkubation des Proteins nach Anspruch 1 mit der zu testenden Substanz,
b) Detektion der Bindung der 'zu testenden Substanz an das Protein durch Messen der thermodynamischen Stabilisierung der Proteinstruktur.

16. Verfahren nach Anspruch 15, dadurch gekennzeichnet daß die Detektion der Bindung durch Messen der Resistenz des Proteins gegen Proteaseabbau in An- und Abwesenheit der zu testenden Substanz durchgeführt wird.

17. Verfahren zum qualitativen und quantitativen Nachweis einer Nukleinsäure nach Anspruch 3 in einer biologischen Probe, das folgende Schritte umfaßt:
a) Inkubation einer biologischen Probe mit einer bekannten Menge an Nukleinsäure gemäß Anspruch 3 oder einer bekannten Menge an Oligonukleotiden, die als Primer für eine Amplifikation der Nukleinsäure gemäß Anspruch 3 geeignet sind,
b) Nachweis der Nukleinsäure gemäß Anspruch 3 durch spezifische Hybridisierung oder PCR-Amplifikation,
c) Vergleich der Menge an hybridisierender Nukleinsäure gemäß Anspruch 3 oder an durch PCR Amplifikation gewonnener Nukleinsäure gemäß Anspruch 3 mit einem Standard.

18. Verfahren zum qualitativen und quantitativen Nachweis eines Proteins gemäß Anspruch 1 in einer biologischen Probe, das folgende Schritte umfaßt:
a) Inkubation einer biologischen Probe mit einem Antikörper, der spezifisch gegen das Protein gemäß Anspruch 1 gerichtet ist,
b) Nachweis des Antikörper/Antigenkomplexes,
c) Vergleich der Mengen des Antikörper/Antigenkomplexes mit einem Standard.
